# EUROPÄISCHE PATENTANMELDUNG

(11) **EP 4 696 406 A1**
(43) Veröffentlichungstag der Anmeldung: **18.02.2026**
(21) Anmeldenummer: 24194250.7
(22) Anmeldetag: 13.08.2024
(51) Int. Cl.: B01F 31/441, B01F 35/71, B01F 35/75, B01F 35/92, A61B 17/88, B65D 81/34, B01F 101/20, B01F 35/90

(54) **VORRICHTUNG ZUR ERWÄRMUNG EINER KOMPONENTE ZUR HERSTELLUNG VON KNOCHENZEMENT UND VERFAHREN**

(71) Anmelder: Heraeus Medical GmbH, 61273 Wehrheim (DE)
(72) Erfinder: Vogt, Sebastian, 61273 Wehrheim (DE); kluge, Thomas, 61273 Wehrheim (DE); Holzapfel, Oliver, 61273 Wehrheim (DE)
(74) Vertreter: Heraeus IP

(57) **Zusammenfassung**

Die Erfindung betrifft ein Verfahren zur Herstellung von Knochenzement sowie eine Vorrichtung zur Erwärmung einer Komponente zur Herstellung von Knochenzement. Bei einem Verfahren zur Herstellung von Knochenzement werden zwei Komponenten (1, 2) miteinander in Kontakt gebracht und gemischt, um Knochenzement herzustellen. Es wird mindestens eine Komponente (2) der zwei Komponenten (1, 2) oder der hergestellte Knochenzement erwärmt. Eine Vorrichtung (10) zur Erwärmung einer Komponente (2) zur Herstellung von Knochenzement umfasst einen Erwärmungsraum (12) zur Aufnahme der Komponente (2) sowie eine Wärmequelle (20) zum Erwärmen der in dem Erwärmungsraum (12) befindlichen Komponente (2). Dies ermöglicht eine beschleunigte Aushärtung und damit eine Zeitersparnis bei der Verwendung von Knochenzement.

## Beschreibung

Die Erfindung betrifft ein Verfahren zur Herstellung von Knochenzement sowie eine Vorrichtung zur Erwärmung einer Komponente zur Herstellung von Knochenzement.

Knochenzement wird üblicherweise durch Mischen eines Pulvers mit einer Flüssigkeit hergestellt. Beispielsweise sind Polymethylmethacrylat-(PMMA)-Knochenzemente bekannt, die aus einer flüssigen Monomerkomponente und einer Pulverkomponente zusammengesetzt sind. Beim Vermischen der Pulverkomponente mit der Monomerkomponente entsteht, z. B. durch Quellung von Polymeren der Pulverkomponente im Methylmethacrylat, ein plastisch verformbarer Teig, der eigentliche Knochenzement (auch als Knochenzementteig bezeichnet). Mit fortschreitender Polymerisation des Methylmethacrylates erhöht sich die Viskosität des Knochenzements, bis dieser erstarrt.

Knochenzement wird beispielsweise verwendet, um Gelenkendoprothesen im knöchernen Implantatlager zu fixieren. Beispielsweise können primäre Gelenkendoprothesen oder Revisionsgelenkendoprothesen zementiert werden, Spacer oder Spacerkomponenten hergestellt werden, etwa für die Interimsphase von zweizeitigen septischen Revisionen von Gelenkendoprothesen, oder frakturierte Wirbelkörper mit Knochenzement aufgefüllt werden. Die oben genannten Merkmale können beliebig mit den Aspekten der Erfindung kombiniert werden.

Bekannte, insbesondere hochviskose Knochenzemente haben bei Raumtemperatur unter Laborbedingungen ein zeitliches Verarbeitungsfenster von ungefähr 3,5 Minuten und härten dann innerhalb von weiteren ca. 3 Minuten exotherm aus. Das Verarbeitungsverhalten von Knochenzement, insbesondere von Polymethylmethacrylat-Knochenzement, ist stark temperaturabhängig. Unter Operations- (OP)-Bedingungen kann die Aushärtung deutlich länger dauern.

OP-Zeit ist teuer und bewegt sich im Bereich von ungefähr 50 bis 100 Euro pro Minute. Eine Einsparung von OP-Zeit ist daher auch aus Kostengründen geboten. Es besteht deshalb Bedarf, die Zeit für die Aushärtung von Knochenzement zu verkürzen, um unnötige Wartezeiten zu verringern.

Ein Lösungsansatz besteht darin, die Zementzusammensetzung so zu ändern, dass diese zu einer schnelleren Aushärtung führt. Ein Beispiel dafür ist der Zement "PALACOS fast R+G" der Heraeus Medical GmbH. Die Verwendung eines solchen Zementes erfordert allerdings Erfahrung und bedarf einer geübten, schnellen Applikation des Zements durch den medizinischen Anwender.

Die Aufgabe der Erfindung ist es, eine beschleunigte Aushärtung von Knochenzement auf besonders einfache Weise zu ermöglichen.

Die Aufgabe wird gelöst durch das Verfahren zur Herstellung von Knochenzement gemäß Anspruch 1 sowie die Vorrichtung zur Erwärmung einer Komponente zur Herstellung von Knochenzement gemäß dem nebengeordneten Anspruch. Vorteilhafte Ausgestaltungen sind in den Unteransprüchen angegeben.

Zur Lösung der Aufgabe dient ein Verfahren zur Herstellung von Knochenzement, bei dem zwei Komponenten miteinander in Kontakt gebracht und gemischt werden, um Knochenzement herzustellen. Mindestens eine der zwei Komponenten oder der hergestellte Knochenzement wird erwärmt.

Sowohl das Erwärmen einer oder beider Komponenten als auch das Erwärmen des Knochenzements hat zur Folge, dass der Knochenzement bei bzw. nach der Applikation eine erhöhte Temperatur aufweist und in der Folge schneller erhärtet. Auf diese Weise ist es möglich, marktübliche Knochenzemente, beispielsweise Polymethylmethacrylat-Knochenzemente, ohne Veränderung der chemischen Zusammensetzung schneller aushärten zu lassen, um wertvolle OP-Zeit zu sparen. Beispielsweise kann dies während der Implantation von Gelenkendoprothesen erfolgen. Das Erwärmen ist ein Beschleunigungsimpuls, der wahlweise von außen eingebracht werden kann. Der medizinische Anwender kann so im Einzelfall entscheiden, ob der relativ langsam aushärtende Zement in gewohnter Weise verwendet werden soll oder ob bei Bedarf eine beschleunigte Aushärtung erfolgen soll.

Die zwei Komponenten sind insbesondere ein Pulver und eine Flüssigkeit. In einer Ausführungsform wird zumindest die Flüssigkeit erwärmt. Dies ist aufgrund der besseren Wärmeleitfähigkeit und Wärmeübertragung der Flüssigkeit effektiver als das Erwärmen des Pulvers. Die Komponenten sind insbesondere so beschaffen, dass eine Aushärtung des Knochenzements erfolgt, insbesondere durch Polymerisation. Bevorzugt erfolgt das Mischen und ggf. auch das Inkontaktbringen der Komponenten in einem Mischbehälter. Der Mischbehälter kann Teil einer Mischvorrichtung sein. Typischerweise wird der Knochenzement anschließend aus dem Mischbehälter ausgetragen, um am Bestimmungsort appliziert zu werden. Das Erwärmen erfolgt insbesondere vor dem Applizieren des Knochenzements. Sowohl das Erwärmen einer oder beider Komponenten vor dem Mischen als auch das Erwärmen des Knochenzements nach dem Mischen führt zu einer beschleunigten Aushärtung.

Das Erwärmen einer Komponente kann in einem Erwärmungsraum und/oder vor dem Einbringen der Komponente in den Mischbehälter erfolgen. Das Erwärmen einer Komponente hat grundsätzlich den Vorteil, dass kein zusätzlicher Schritt während der Reaktionszeit der Komponenten benötigt wird. So kann das Knochenzement nach der Herstellung wie gewohnt und ohne zusätzlichen Zeitdruck verarbeitet werden.

In einer Ausgestaltung befindet sich ein Behälter, der eine Komponente enthält, während des Erwärmens in einem Erwärmungsraum. Ein Erwärmungsraum ist ein Hohlraum, in dem die Erwärmung erfolgen kann. In dieser Ausgestaltung ist der Erwärmungsraum dazu eingerichtet, den Behälter mit der Komponente aufzunehmen. Der Erwärmungsraum kann Teil einer Vorrichtung zur Erwärmung sein. Es kann eine Wärmequelle vorgesehen sein, insbesondere angrenzend an den Erwärmungsraum, um eine Substanz und/oder einen Gegenstand, die oder der sich im Erwärmungsraum befindet, zu erwärmen. Der Behälter kann offen oder geschlossen sein.

Insbesondere wird der Behälter mit der Komponente vor der Erwärmung in den Erwärmungsraum eingeführt. Nach erfolgter Erwärmung kann die Komponente direkt aus dem im Erwärmungsraum befindlichen Behälter entnommen werden. Ein Entnehmen des Behälters aus dem Erwärmungsraum ist nicht notwendig. Es ist allerdings auch nicht ausgeschlossen, dass der Behälter aus dem Erwärmungsraum entnommen wird.

Das OP-Personal kann intraoperativ, beispielsweise unmittelbar vor einer Implantation von zementierten Gelenkendoprothesen, eine Komponente wie z. B. Monomerflüssigkeit innerhalb von wenigen Sekunden bis Minuten beispielsweise auf eine Temperatur von mindestens 40 °C erwärmen. Die Aushärtung des Polymethylmethacrylat-Knochenzementteigs kann durch die Erwärmung um mindestens 2 Minuten beschleunigt werden, um OP-Zeit einzusparen.

In einer Ausgestaltung wird der Behälter von einer Halteeinrichtung im Erwärmungsraum gehalten. Insbesondere wird die Halteeinrichtung mit dem Behälter nach dem Erwärmen aus dem Erwärmungsraum entnommen und/oder mechanisch mit einer Mischvorrichtung gekoppelt. Dies kann erfolgen, um den Behälter zu öffnen.

Hierbei wird der Behälter, der insbesondere eine Ampulle ist, auf definierte Weise geöffnet. Dabei befindet sich der Behälter bevorzugt in einer Öffnungsvorrichtung, die so mit der Mischvorrichtung verbunden ist oder verbunden werden kann, dass die Komponente aus dem Behälter in die Mischvorrichtung überführt werden kann. Insbesondere ist die Öffnungsvorrichtung mechanisch mit der Mischvorrichtung verbunden. Insbesondere ist zwischen der Öffnungsvorrichtung und der Mischvorrichtung ein Fluidkanal zum Transport der Komponente vorgesehen. Auf diese Weise kann das Überführen des Behälters aus dem Erwärmungsraum in die Mischvorrichtung besonders einfach und definiert erfolgen.

Die Halteeinrichtung wird mit der Mischvorrichtung gekoppelt. Beispielsweise wird die Halteeinrichtung in einen Bereich der Mischvorrichtung eingeführt, beispielsweise in die Öffnungsvorrichtung.

Die Mischvorrichtung kann als Kartusche ausgeführt sein. Die Mischvorrichtung kann einen Innenraum zum Mischen umfassen. In dem Innenraum kann die andere Komponente zur Herstellung von Knochenzement gelagert sein, insbesondere die Pulverkomponente. Die Mischvorrichtung kann ferner ein im Innenraum befindliches Mischelement umfassen, das beispielsweise mit einem Handgriff für ein manuelles Mischen verbunden ist. Die Öffnungsvorrichtung kann durch die Halteeinrichtung und/oder die Mischvorrichtung gebildet sein.

Die Halteeinrichtung hält den Behälter. Es können zwei oder mehr Behälter mit einer Komponente von der Halteeinrichtung gehalten werden. Diese können dann gleichzeitig erwärmt, entnommen und/oder geöffnet werden. Die Halteeinrichtung kann den Behälter so halten, dass die Wandung des Behälters zumindest teilweise freiliegt. Auf diese Weise kann die Wärmeübertragung in den Behälter besonders effektiv erfolgen, wenn der Behälter von der Halteeinrichtung gehalten wird. Die Halteeinrichtung kann einen Griffbereich zum manuellen Greifen aufweisen.

Die Halteeinrichtung kann den Behälter im Inneren der Öffnungsvorrichtung halten. Die Halteeinrichtung kann dazu eingerichtet sein, gemeinsam mit dem Behälter relativ zu einem Scherelement zum Abscheren des Kopfes einer Ampulle bewegt zu werden, so dass die Ampulle geöffnet werden kann. die Halteeinrichtung kann hohlzylinderförmig sein.

Die Halteeinrichtung kann Teil eines Prepacked-Mischsystems sein, das alle für das Herstellen und Auspressen von Knochenzement benötigten Stoffe und Vorrichtungen enthält. Dies erleichtert die Handhabung des Behälters. Der Behälter kann in der Halteeinrichtung gelagert sein. Die Halteeinrichtung mit dem Behälter kann in der Öffnungsvorrichtung gelagert sein. Dies entspricht der üblichen Anordnung bei Prepacked-Mischsystemen. Erfindungsgemäß kann nun die Halteeinrichtung mit dem Behälter in die Vorrichtung zum Erwärmen überführt werden. Es wird ermöglicht, auch eine Komponente eines Prepacked-Mischsystems auf einfache Weise zu erwärmen. Alternativ kann die Halteeinrichtung mit dem Behälter in dem Erwärmungsraum gelagert sein. Das Verfahren kann das Einsetzen der Halteeinrichtung mit dem darin gehaltenen Behälter in den Erwärmungsraum umfassen.

Ein weiterer Aspekt der Erfindung ist eine Vorrichtung zur Erwärmung einer Komponente zur Herstellung von Knochenzement. Die Vorrichtung umfasst einen Erwärmungsraum zur Aufnahme der Komponente sowie eine Wärmequelle zum Erwärmen der in dem Erwärmungsraum befindlichen Komponente. Alle Vorteile, Merkmale, und Ausgestaltungen des eingangs beschriebenen Verfahrens können analog für die Vorrichtung gelten und umgekehrt.

Die Vorrichtung ermöglicht auf einfache Weise eine Erwärmung der Komponente. Die Komponente dient der Herstellung von Knochenzement und ist beispielsweise eine flüssige Komponente. Wie beschrieben führt das direkt zu einer beschleunigten Aushärtung. Die Vorrichtung kann selbstverständlich auch dazu eingerichtet sein, den hergestellten Knochenzement im Erwärmungsraum aufzunehmen und zu erwärmen.

Der Erwärmungsraum ist ein Hohlraum, in dem die Komponente angeordnet werden kann. Der Erwärmungsraum kann zum Großteil oder im Wesentlichen verschlossen sein oder werden. Typischerweise besteht ein Zugang von außen in den Erwärmungsraum, durch den die Komponente in den Erwärmungsraum eingebracht werden kann. Der Zugang kann im Wesentlichen oder vollständig dicht verschließbar sein. Der Erwärmungsraum kann derart dicht sein oder abgedichtet werden, dass die Komponente im flüssigen oder pulverförmigen Zustand eingefüllt werden kann. Es ist jedoch bevorzugt, dass ein typischerweise geschlossener Behälter mit der Komponente in dem Erwärmungsraum angeordnet wird. In diesem Fall ist eine vollständig dichte Ausführung nicht zwingend notwendig.

Die Wärmequelle steht typischerweise mit dem Erwärmungsraum in thermischer Verbindung. Im einfachsten Fall grenzt die Wärmequelle direkt oder indirekt an den Erwärmungsraum an, so dass Wärme der Wärmequelle über einen kurzen Weg an die im Erwärmungsraum befindliche Komponente übertragen werden kann. Dabei kann die Wärme beispielsweise durch eine Wandung des Behälters, in dem sich die Komponente befindet, übertragen werden.

Insbesondere umfasst die Vorrichtung ein Gehäuse, in welchem der Erwärmungsraum angeordnet bzw. ausgebildet ist. Die Vorrichtung kann zum einmaligen Gebrauch vorgesehen sein. Auf diese Weise können die hohen hygienischen Anforderungen bei gleichzeitig einfacher Konstruktion erfüllt werden.

Die Vorrichtung kann die Komponente umfassen, insbesondere der Behälter mit der Komponente. Der Behälter kann beispielsweise ein Beutel sein, z. B. aus Kunststoff oder einem Kunststoffverbundmaterial, der mit Metall beschichtet sein kann, oder eine Ampulle, z. B. aus Glas oder Kunststoff. Besonders bevorzugt sind Glasampullen, die eine Lagerung der Flüssigkeit verlustfrei und über mehrere Jahre bei Raumtemperatur ermöglichen und zudem kostengünstig herstellbar sind.

In einer Ausgestaltung umfasst die Wärmequelle einen Wärmespeicher, der nach Aktivierung Wärme abgeben kann. Der Wärmespeicher ist insbesondere ein thermochemischer Wärmespeicher.

Ein Wärmespeicher ist eine Einrichtung, die gespeicherte Energie enthält und diese in Form von Wärme bei Aktivierung abgeben kann. Diese Ausgestaltung ermöglicht, dass die Wärmefreisetzung besonders einfach ausgelöst werden kann und ohne Batterien oder Akkumulatoren sowie ohne Verbrennung erfolgt. Es wird kein Brennstoff benötigt. Es ist möglich, die Wärmefreisetzung innerhalb von Sekunden zu aktivieren. Zudem kann durch die geeignete Wahl der Größe und Art des Wärmespeichers bzw. der Menge des enthaltenen Materials oder der enthaltenen Materialien, insbesondere im Verhältnis zur Menge des zu erwärmenden Stoffes, eine geeignete Zieltemperatur und/oder eine geeignete Erwärmungsdauer eingestellt werden. Beispielsweise kann gewünscht sein, dass die Wärmefreisetzung nur innerhalb weniger Minuten abläuft und danach sicher beendet ist.

Insbesondere ist der Wärmespeicher so eingerichtet, dass eine Monomerflüssigkeit auf eine Temperatur von mindestens 40° C, bevorzugt mindestens 50° C, besonders bevorzugt mindestens 60° C erwärmt wird. So kann eine besonders effektive Verkürzung der Zeit zum Aushärten erreicht werden. Insbesondere erfolgt die Erwärmung auf höchstens 90° C, bevorzugt höchstens 85° C, besonders bevorzugt höchstens 80° C oder höchstens 75° C. Eine zu hohe Temperatur kann zu einem starken Verdunsten bzw. Verdampfen des Monomers bis hin zum Sieden führen und ist deshalb ungewünscht. Beispielsweise siedet das typische Monomer für PMMA-Knochenzement bei 101 °C.

Als thermochemischer Wärmespeicher werden Systeme bezeichnet, die Wärme durch eine gezielt aktivierbare exotherme Reaktion und/oder eine Sorptionsreaktion abgeben können. Insbesondere enthält der thermochemische Wärmespeicher ein geeignetes Wärmespeichermedium, das nach Aktivierung Wärme freisetzt. Insbesondere umfasst der thermochemische Wärmespeicher einen Raum, in dem sich das Wärmespeichermedium befindet.

Das Wärmespeichermedium ist insbesondere ein Feststoff und/oder reagiert exotherm mit zugegebenem Wasser. Reagieren bzw. Reaktion meint hierbei nicht notwendigerweise eine chemische Reaktion. Beispielsweise wird im Sinne der Erfindung auch eine Adsorption als Reaktion bezeichnet. Das Wärmespeichermedium ist insbesondere ein anorganische Verbindung oder eine Mischung mit oder aus anorganischen Verbindungen. Das Wärmespeichermedium kann wasserlöslich, in Wasser teilweise löslich oder wasserunlöslich sein. Insbesondere ist das Wärmespeichermedium so ausgewählt, dass bei der Reaktion zur Wärmfreisetzung kein Gas freigesetzt wird.

Neben thermochemischen Wärmespeichern können grundsätzlich auch Latentwärmespeicher verwendet werden. Diese sind allerdings nicht transportstabil, da bei Erschütterungen eine ungewünschte Aktivierung erfolgen kann. Dies ist bei thermochemischen Wärmespeichern nicht der Fall, die bei dichter Lagerung, die ein Eindringen von Wasser ausschließt, transportstabil sind.

In einer Ausgestaltung ist der thermochemische Wärmespeicher so beschaffen, dass er durch Zugabe von Wasser aktiviert werden kann. Zur Aktivierung muss nicht notwendigerweise reines Wasser verwendet werden. Ebenso kann typischerweise eine wässrige Lösung, beispielsweise eine Kochsalzlösung verwendet werden. Wasser oder wässrige Lösungen sind in jedem Operationssaal vorhanden, so dass dies eine einfache und überall funktionierende Möglichkeit zur Aktivierung bereitstellt. Zudem ist in dieser Ausgestaltung eine unbeabsichtigte Wärmefreisetzung während der Lagerung oder des Transports der Vorrichtung ausgeschlossen.

Die Vorrichtung umfasst also neben dem Erwärmungsraum einen weiteren Raum. Insbesondere umfasst die Vorrichtung eine Öffnung, über die Wasser in den Raum gegeben werden kann. Insbesondere kann die Öffnung reversibel mit einem Deckel verschlossen werden. Insbesondere enthält die Vorrichtung eine Dichtung, um ein dichtes Verschließen der Öffnung mit dem Deckel zu ermöglichen. Auf diese Weise kann eingefülltes Wasser auch dann nicht wieder entweichen, auch wenn die Vorrichtung bewegt oder gedreht wird.

In einer Ausgestaltung umfasst der thermochemische Wärmespeicher Zeolith, Kieselgel und/oder wasserfreies Calciumchlorid. Bevorzugt ist der enthaltene Stoff (das Wärmespeichermedium) wasserfrei, stark hygroskopisch und besitzt bevorzugt eine große innere Oberfläche. Die genannten Stoffe sind nicht brennbar, nicht explosiv, nicht selbstentzündlich, ökologisch und toxikologisch unbedenklich, kostengünstig, kommerziell gut verfügbar und weisen auch bei langer Lagerung keinen Verlust an Wärmefreisetzung auf. Es kann ein Silicat verwendet werden. Zeolith ist besonders zu bevorzugen, da er besonders einfach verfügbar ist und keine sauren oder basischen Lösungen erzeugt. Zudem beginnt die Wärmefreisetzung unmittelbar nach der Wasserzuführung, so dass eine sehr schnelle Erwärmung möglich ist. Auch nach der Reaktion mit Wasser sind Zeolithe und Kieselgele völlig ungefährlich, haben einen unkritischen pH-Wert und können problemlos entsorgt werden.

Alternativ oder ergänzend kann der thermochemische Wärmespeicher ein Gemisch aus Eisenpulver, Natriumchlorat und Kupfersulfat und/oder aus Eisenpulver, Natriumperchlorat und Kupfersulfat umfassen. Diese Gemische sind im trockenen Zustand lagerstabil. Die Wärmefreisetzung erfolgt erst bei Kontakt der Gemische mit Wasser. Dabei wird das Eisenpulver durch das Natriumchlorat oder Natriumperchlorat zu Eisenoxid oxidiert, wobei Wärme freigesetzt wird. Die maximale Temperatur wird durch das Verhältnis von Eisenpulver zu Chlorat oder Perchlorat bestimmt. Für die Erwärmung auf Temperaturen unter 100 °C können Gemische von Eisenpulver mit Chlorat und Perchlorat verwendet werden, bei denen das Chlorat oder Perchlorat in unterstöchiometrischer Menge im Gemisch vorliegt.

Insbesondere beträgt ein Gewichtsverhältnis des Wärmespeichermediums zu dem zuzufügenden Wasser mindestens 1,0 und/oder höchstens 1,2.

In einer Ausgestaltung weist der thermochemische Wärmespeicher ein Wärmespeichermedium auf, welches in Form von Partikeln vorliegt. Die Partikel haben insbesondere einen Durchmesser von mindestens 0,1 mm und/oder höchstens 4 mm.

Der Durchmesser ist bevorzugt mindestens 0,2 mm, besonders bevorzugt 0,5 mm oder 1 mm. Zu feine Partikel erschweren das Eindringen des Wassers, so dass die Erwärmung ungleichmäßig und/oder verzögert erfolgt. Der Durchmesser ist bevorzugt höchstens 4 mm, besonders bevorzugt höchstens 3 mm und insbesondere höchstens 2 mm. Zu große Partikel verringern die Kontaktfläche und verzögern auf diese Weise die Erwärmung. Die Partikel können als Granulat, Kugeln und/oder Stäbchen vorliegen.

In einer Ausführungsform enthält der thermochemische Wärmespeicher zudem einen Superabsorber, insbesondere in Form von Partikeln. Dies bindet das zugeführte Wasser und verhindert ein Ausschwappen oder Auslaufen.

In einer Ausgestaltung umfasst die Vorrichtung ferner einen Temperaturindikator zur optischen Anzeige einer definierten Temperatur im Inneren der Vorrichtung. Auf diese Weise kann einfach geprüft werden, ob die Wärmefreisetzung und die Temperierung der Monomerflüssigkeit begonnen hat bzw. ob die Temperatur im gewünschten Bereich ist, beispielsweise oberhalb von 60° C. Der Temperaturindikator kann eine thermochrome Substanz und/oder z. B. thermochrome Flüssigkristalle aufweisen. Bei Erreichen einer definierten Temperatur ändert der Temperaturindikator dann seine Farbe. Insbesondere ist der Temperaturindikator an der Oberseite der Vorrichtung angebracht bzw. von der Oberseite aus sichtbar.

In einer Ausgestaltung ist zumindest eine umlaufende Außenseite der Vorrichtung thermisch isoliert ist. Die Vorrichtung kann einen runden und/oder eckigen Querschnitt aufweisen und/oder länglich geformt sein. Die umlaufende Außenseite meint die Seite, die die Vorrichtung in Umfangsrichtung begrenzt. Diese kann im Falle eines runden Querschnitts eine Mantelfläche sein und im Falle eines eckigen Querschnitts aus mehreren, z. B. vier Flächen zusammengesetzt sein. Die Vorrichtung weist zudem insbesondere eine Unterseite, z. B. zum Aufstellen der Vorrichtung auf einen Grund, und/oder eine Oberseite auf, beispielsweise mit dem Zugang zum Erwärmungsraum und/oder der Öffnung des Raums. Insbesondere ist auch die Unterseite thermisch isoliert.

Die thermische Isolierung kann ein Wärmedämmmaterial aufweisen, beispielsweise einen Schaum, etwa einen Kunststoffschaum. Alternativ oder ergänzend kann die thermische Isolierung durch eine doppelwandige Konstruktion der Außenwand realisiert sein, wobei sich zwischen zwei Wänden ein Luftraum befindet, der ggf. einen Unterdruck aufweisen kann.

Die thermische Isolierung verringert Wärmeverluste und sorgt so für ein schnelleres Aushärten. Zudem wird die Handhabung der Vorrichtung im heißen Zustand angenehmer und sicherer, da eine Verbrennungsgefahr ausgeschlossen werden kann.

In einer Ausgestaltung weist eine Wandung, die den thermochemischen Wärmespeicher von dem Erwärmungsraum trennt, eine oder mehrere Durchgangsöffnungen auf. Die Durchgangsöffnungen sind so beschaffen, dass Flüssigkeit wie z. B. Wasser aus dem Raum des thermochemischen Wärmespeichers in den Erwärmungsraum gelangen kann. Der Behälter mit der Komponente kann dann in direkten Kontakt mit der erwärmten Flüssigkeit gelangen. Die Wärmeübertragung wird so weiter verbessert und beschleunigt. Insbesondere sind mindestens 6 oder mindestens 10 oder mindestens 15 Durchgangsöffnungen vorhanden.

Insbesondere ist im Bereich der Öffnung zum Einsetzen des Behälters ein Dichtungselement vorhanden, z. B. in Form eines O-Rings, um den Erwärmungsraum gegenüber dem eingesetzten Behälter abzudichten, wobei der Behälter insbesondere eine Ampulle ist. So wird verhindert, dass Flüssigkeit aus dem Inneren des Erwärmungsraums nach außen gelangt und dass die Komponente beim Ausgeben kontaminiert wird.

In einer Ausgestaltung ist eine Außenwand im Bereich des thermochemischen Wärmespeichers zumindest bereichsweise transparent oder transluzent. Dies ist insbesondere so realisiert, dass durch die transparente oder transluzente Außenwandung hindurch eine optische Kontrolle eines Füllstands einer zugefügten Flüssigkeit möglich ist. So kann der Anwender einfach sehen, wann ausreichend Flüssigkeit wie z. B. Wasser hinzugefügt wurde.

Insbesondere enthält der thermochemische Wärmespeicher zudem einen Farbstoff. Dies erleichtert die optischen Kontrolle des Füllstands. Insbesondere befindet sich in dem Raum ein in Wasser löslicher, nicht toxischer Farbstoff. Bevorzugt ist ein grüner und/oder blauer Farbstoffe vorhanden. Besonders bevorzugt ist ein Lebensmittelfarbstoff. Beispielsweise können einer oder mehrerer der folgenden Farbstoffe verwendet werden: Methylenblau (CAS 61-73-4), Indigocarmin (CAS 860-22-0), Grün S (CAS 3087-16-9), Patentblau V (CAS 20262-76-4) und wasserlösliches Chlorophyllin (11006-34-1).

In einer Ausführungsform weist die Vorrichtung eine Abdeckung, z. B. eine Abdeckklappe, zum Verschließen des Zugangs zum Einführen des Behälters auf. Die Abdeckung kann thermisch isoliert sein. So können Wärmverluste weiter verhindert werden. Die Abdeckung kann den Zugang reversibel verschließen.

In einer Ausgestaltung weist der thermochemische Wärmespeicher eine Entlüftungsöffnung auf, um beim Zugeben von Wasser verdrängte Luft ausströmen zu lassen. Insbesondere ist die Entlüftungsöffnung mit einem Filterelement verschlossen, beispielsweise durch ein Sieb, Netz oder eine Porenscheibe, um ein Austreten von Wärmespeichermedium zu verhindern. Es kann ein Deckel vorhanden sein, um die Entlüftungsöffnung reversibel zu verschließen.

In einer Ausgestaltung weist ein Raum des thermochemischen Wärmespeichers zwei Bereiche auf. Ein erster Bereich dient zum Zugeben des Wassers. Ein zweiter Bereich enthält ein Wärmespeichermedium. Der erste Bereich und der zweite Bereich sind lediglich im Bereich einer unteren Begrenzung des Raums miteinander verbunden.

Insbesondere enthält der erste Bereich kein Wärmespeichermedium und/oder ist mit Luft gefüllt. Der erste Bereich und der zweite Bereich sind weitgehend voneinander getrennt und nur im Bereich der unteren Begrenzung miteinander verbunden. Die unteren Begrenzung ist die Begrenzung des Raums, die der Oberseite abgewandt ist, an der die Öffnung zum Zugeben von Wasser abgewandt ist. Der Bereich der unteren Begrenzung weist in Längsrichtung des Raums bzw. der Vorrichtung eine Erstreckung von maximal 20% der Gesamthöhe des Raums auf. Die Verbindung muss sich also nicht notwendigerweise unmittelbar an der unteren Begrenzung befinden.

Das Wasser muss also zunächst den ersten Bereich vollständig durchströmen, um in den zweiten Bereich zu gelangen. Auf diese Weise wird das Wärmespeichermedium von unten befeuchtet. Entstehender Dampf gelangt so in den Raum mit dem Wärmespeichermedium. Es wird verhindert, dass beim Befüllen Dampf aus der Öffnung austritt.

Der erste Bereich kann klein im Vergleich zum zweiten Bereich sein. Der erste Bereich kann als beispielsweise rohrförmiges Einlaufelement im zweiten Bereich angeordnet sein. das Einlaufelement kann eine dichte Wand aufweisen, die lediglich im Bereich der unteren Begrenzung durchbrochen bzw. geöffnet ist oder fehlt. Eine etwaig vorhandene Entlüftungsöffnung ist insbesondere am zweiten Bereich angeordnet.

In einer Ausgestaltung weist die Vorrichtung eine Halteeinrichtung zum Halten des Behälters im Erwärmungsraum auf. Die Halteeinrichtung ist zum mechanischen Koppeln mit einer Mischvorrichtung eingerichtet. Die Mischvorrichtung dient dem Mischen zweier Komponenten zwecks Herstellung von Knochenzement und/oder ist ein separater Gegenstand.

Ein unabhängiger Aspekt der Erfindung ist ein System, umfassend eine erfindungsgemäße Vorrichtung sowie eine Mischvorrichtung zum Mischen zweier Komponenten zwecks Herstellung von Knochenzement.

In einer weiteren Ausgestaltung der Vorrichtung ist ein Gehäuse der Vorrichtung zumindest im Wesentlichen durch Kunststoffspritzgießen hergestellt. Im Wesentlichen meint, dass zumindest wesentliche Teile des Gehäuses durch Kunststoffspritzgießen hergestellt sind. Anschließend können diese Teile, ggf. mit anders hergestellten Teilen, verbunden worden sein. Das Gehäuse bildet den Erwärmungsraum und insbesondere ein Außengehäuse der Vorrichtung und/oder einen Raum zur Aufnahme des Wärmespeichermediums aus. Auf diese Weise ist eine einfache und kostengünstige Herstellung möglich.

Alternativ oder ergänzend besteht die Vorrichtung zumindest überwiegend aus thermoplastischem Kunststoff. Alternativ oder ergänzend ist die Vorrichtung so ausgestaltet, dass sie problemlos mit dem üblichen Klinikabfall entsorgt werden kann.

Ein erfindungsgemäßes Verfahren, insbesondere unter Verwendung einer erfindungsgemäßen Vorrichtung, kann einen oder mehrere der folgenden Schritte in beliebiger Kombination umfassen:
- Ggf. Öffnen einer Abdeckung, um den Zugang zum Erwärmungsraum freizugeben,
- Einbringen des Behälters in den Erwärmungsraum, insbesondere über den Zugang,
- Ggf. Schließen der Abdeckung,
- Öffnen des Deckels,
- Einfüllen von Wasser, um die Erwärmung zu aktivieren,
- Ggf. Schließen des Deckels,
- Insbesondere nach erfolgter Erwärmung: ggf. Öffnen der Abdeckung,
- Öffnen des Behälters zum Entnehmen der Komponente,
- Entnahme der Komponente.

Ein weiterer Aspekt der Erfindung ist die Verwendung einer insbesondere auf über 40 C erwärmten Komponente zur Herstellung von Knochenzement.

### Beispiele

Zur Prüfung des Verarbeitungsverhaltens von Polymethylmethacrylat-Knochenzement wird üblicherweise mit den Händen eine Kugel aus Zementteig geformt und die Verformbarkeit dieser Kugel wird in Abhängigkeit von der Zeit manuell geprüft. Die Aushärtung der Kugel wird durch Klopfen der Kugel akustisch bestimmt. Die vollständige Aushärtung kann durch einen hellen Klang des Klopfgeräuschs erkannt werden. Die zu prüfende Kugel hat bei einer Zementpulvermenge von 40 g einen Durchmesser von ungefähr 4,5 cm. Aufgrund der Kugelform ist das Verhältnis der Oberfläche zum Volumen der Zementkugel klein. Die Polymerisation startet zwar im gesamten Volumen der Kugel etwa gleichmäßig, beschleunigt sich jedoch im Inneren der Kugel selbst massiv infolge eines Wärmestaus durch die freigesetzte Polymerisationswärme. Die im Inneren freigesetzte Wärme kann nur langsam nach außen abgeführt werden. Dadurch erhöht sich im Inneren der Kugel die Temperatur, wodurch die Polymerisationsgeschwindigkeit dort massiv zunimmt. Zudem wird bereits beim Herstellen der Kugel durch die Hände eine gewisse Wärmeenergie in den Knochenzement eingebracht. Unter diesen Bedingungen benötigt ein Knochenzement wie Heraeus Palacos ungefähr 6 bis 6,5 Minuten zur Aushärtung.

In der OP-Praxis zeigt sich, dass die Aushärtung deutlich später als unter Laborbedingungen erfolgt. Heraeus Palacos benötigt hier ungefähr 11 bis 13 Minuten, Knochenzemente anderer Hersteller zum Teil noch länger. Dies ist unter anderem darauf zurückzuführen, dass der Polymethylmethacrylat-Knochenzement dabei als dünne Schicht mit einer Schichtdicke im Bereich weniger Millimeter vorliegt, beispielsweise an der Oberfläche einer Prothese. Das bedeutet, dass das Verhältnis von Zementoberfläche zum Zementvolumen deutlich größer ist als bei Zementkugeln und die entstehende Wärme so besser an die Umgebung abgegeben wird. Zudem bestehen Gelenkendoprothesen üblicherweise aus Chrom-Kobalt-Stahl bestehen und weisen somit eine gute Wärmeleitfähigkeit und hohe Wärmekapazität auf. Dadurch wird der Zementteig sehr wirksam durch die anliegende Gelenkendoprothese gekühlt und die Aushärtung setzt später ein bzw. erfolgt langsamer als bei einer Kugel aus Zementteig unter Laborbedingungen.

Da die Verarbeitungszeit durchschnittlich 3 bis 3,5 Minuten beträgt, fällt bis zum Aushärten des Knochenzements noch eine lange Wartezeit im Bereich von 8 bis 10 Minuten an. Diese kann erfindungsgemäß um 3 bis 3,5 Minuten verkürzt werden.

### Beispiel 1 - Laborbedingungen

Zuerst wurde die Aushärtung von PMMA-Knochenzement in Abhängigkeit von der Temperatur der eingesetzten Monomerflüssigkeit bestimmt. Dazu wurden jeweils 40,8 g PMMA-Knochenzementpulver PALACOS^{®} R+G, das auf Raumtemperatur temperiert war, mit jeweils 20 ml Monomerflüssigkeit, die zuvor auf Raumtemperatur, auf 40 °C, auf 50 °C, auf 60 °C und auf 70 °C temperiert wurde, in einer Mischschüssel mit Hilfe eines Spatels vermischt. Die Bestimmung des Verarbeitungs- und Aushärtungsverhaltens des gemischten PMMA-Knochenzementteigs erfolgte mit einer mit den Händen hergestellten Zementkugel von ungefähr 4,5 cm Durchmesser in Anlehnung an die ISO5833.

Die Ergebnisse sind in der nachfolgenden Tabelle 1 angegeben. Dargestellt sind der Beginn der Verarbeitungsphase, das Ende der Verarbeitungsphase sowie die Dauer der Aushärtung, jeweils in Sekunden bzw. Minuten ab dem Ende des Mischens. Der Beginn der Verarbeitungsphase ist gekennzeichnet durch die Klebfreiheit. Das Ende der Verarbeitungsphase ist gekennzeichnet durch einen Übergang vom plastisch verformbaren Verhalten zum elastischen Verhalten. Dies kann ermittelt werden durch Falten einer aus der Kugel hergestellten Platte. Sobald die Platte beginnt, sich aufzustellen, ist die Verarbeitungsphase beendet. Das Ende der Aushärtung wird wie oben beschrieben akustisch geprüft.

**Tabelle 1**

| **Temperatur der Monomerflüssigkeit** | **Beginn der Verarbeitungsphase** | **Ende der Verarbeitungsphase** | **Aushärtung** |
|---|---|---|---|
| Raumtemperatur | 45 s | 5 min 00 s | 7 min 10 s |
| 40 °C | 40 s | 4 min 20 s | 6 min 25 s |
| 50 °C | 35 s | 4 min 05 s | 6 min 15 s |
| 60 °C | 30 s | 3 min 10 s | 5 min 20 s |
| 70 °C | 30 s | 2 min 50 s | 5 min 00 s |

Die Ergebnisse der Versuche zeigen, dass mit zunehmender Temperatur der Monomerflüssigkeit die Zeit bis zur Aushärtung signifikant sinkt. Wenn die Monomerflüssigkeit statt Raumtemperatur 70° C aufweist, ist unter Laborbedingungen bereits eine Beschleunigung um mehr als 2 Minuten möglich.

### Beispiel 2 - praxisnähere Bedingungen

In der OP-Praxis prüfen die medizinischen Anwender häufig die Aushärtung des PMMA-Knochenzementteig nach der Implantation, in dem sie am freiliegenden Bereich des PMMA-Zementteigs zwischen dem knöchernen Implantatlager und der Prothesenkomponente mit einem Spatel oder einem anderen festen Gegenstand in den Zementteig drücken. Sobald der Spatel nicht mehr in den Zementteig eingedrückt werden kann und beim Klopfen mit dem Spatel auf dem Zementteig ein heller Ton entsteht, wird der Zementteig als ausgehärtet betrachtet.

Ausgehend von dieser Prüfmethode wurde ein Versuchsaufbau zur Simulation der Aushärtung von PMMA-Knochenzement zur Zementierung von Kniegelenk-Endoprothesen entwickelt, um den Einfluss der Temperierung der Monomerflüssigkeit auf die Aushärtungszeit unter praxisnäheren Bedingungen zu bestimmen. Bei der Zementierung von Kniegelenk-Endoprothesen ist die Schichtdicke des Zementteigs üblicherweise im Bereich von ungefähr 3 mm bis 4 mm. Die Tibiakomponente und auch die Femurkomponente haben jeweils eine Masse, je nach Typ und Größe, im Bereich von ungefähr 130 g bis 230 g. Beide Komponenten bestehen normalerweise aus Chrom-Kobalt-Stahl und sind vor der Implantation auf Raumtemperatur temperiert. Das bedeutet, dass die Komponenten Wärmesenken darstellen, welche die bei der Aushärtung des PMMA-Knochenzementes freigesetzte Wärmeenergie aufnehmen. Dadurch wird der PMMA-Knochenzement gekühlt. Die Temperatur des knöchernen Implantatlagers liegt im Bereich von 36 °C bis 37 °C. Zur Simulation dieser Bedingungen wurde folgender Versuchsaufbau entwickelt:
Es wurde eine POM-Kunststoffplatte der Abmessungen 10,0 cm × 10,0 cm × 1,0 cm und einem Gewicht von 153,2 g auf den Labortisch als Isolationsschicht aufgelegt. Darauf wurde eine Edelstahlplatte mit einer Masse von 293,5 g und den Abmessungen 7,8 cm × 4,8 cm × 1,0 cm als Modell einer Komponente von Kniegelenk-Endoprothesen aufgesetzt. Die Edelstahlplatte war auf Raumtemperatur temperiert. Die Masse der Edelstahlplatte war höher als die Masse der typischerweise verwendeten, oben beschriebenen Komponenten der Endoprothesen. Auf diese wurde ein Stahlrahmen mit den äußeren Abmessungen 7,7 cm × 5,4 cm und einer Höhe von 3,45 mm und den inneren Abmessungen von 6,5 cm × 4,2 cm und einer Höhe von 3,45 mm aufgelegt.

Es wurden jeweils 20 ml PALACOS-Monomerflüssigkeit (Lot 1336) auf Raumtemperatur, auf 40 °C, auf 50 °C, auf 60 °C und auf 70 °C temperiert. Jeweils 20 ml der temperierten Monomerflüssigkeit wurde in ein PALAMIXO-Mischsystem gegeben, dann wurde 40,8 g PALACOS^{®} R+G-Zementpulver (Lot 6976) zugefügt. Nach dem Verschließen des Mischsystems wurde zügig manuell gemischt. Der gebildete Zementteig, beispielsweise aufweisend eine Temperatur zwischen 35 °C und 40 °C, wurde anschließend mit einem entenschnabelfömigen Austragsrohr (engl.: "duck nozzle") aus dem Palamix-Mischsystem mit Hilfe einer manuell bedienbaren Auspressvorrichtung in den Rahmen eingebracht. Dann wurde eine Kunststofffolie aufgelegt und eine auf 37°C temperierte HVPC-Plastikplatte mit einer Masse von 144,1 g und den Abmessungen 10,0 cm × 10,0 cm × 1,0 cm auf die Folie gepresst bis der Zementteig eine ebene Oberfläche hatte und bündig zum oberen Rand des Metallrahmens war. Der Zementteig in dem Rahmen hatte damit eine Schichtdicke von 3,45 mm. Die auf 37 °C temperierte Kunststoffplatte wurde zur Simulation des Wärmeeintrags des knöchernen Implantatlagers 4,0 min auf dem Zementteig belassen. Dann wurde im zeitlichen Abstand von 30 Sekunden durch Eindrücken beziehungsweise durch Klopfen mit einem Metallspatel geprüft, ob der Knochenzementteig ausgehärtet ist.

Die Ergebnisse dieses Versuches sind in der folgenden Tabelle 2 dargestellt.

**Tabelle 2**

| **Temperatur der Monomerflüssigkeit** | **Aushärtung** |
|---|---|
| Raumtemperatur | 10 min 00 s |
| 40 °C | 9 min 30 s |
| 50 °C | 8 min 30 s |
| 60 °C | 8 min 00 s |
| 70 °C | 7 min 00 s |

Zunächst ist ersichtlich, dass die Aushärtung unter praxisnäheren Bedingungen länger dauert. Die Ergebnisse zeigen, dass trotz der Kühlung durch die Metallplatte, als Modell der Prothesenkomponente, und des gleichzeitigen Wärmeeintrags durch die auf 37 °C temperierte Kunststoffplatte, als Modell des knöchernen Implantatlagers, eine deutliche Verkürzung der Aushärtungszeit durch Verwendung von erwärmter Monomerflüssigkeit erreicht wird. Es kann eine Verkürzung um 3 Minuten erfolgen.

### Beispiel 3 - Bestimmung der Erwärmung 1

Im Folgenden wurde eine erfindungsgemäße Vorrichtung durch 3D-Druck per selektivem Lasersintern (SLS) aus dem Kunststoff PA12 hergestellt. Die Vorrichtung wurde mit 90 g getrocknetem Zeolith A (Kugeln mit einem Durchmesser von 1,6 mm) befüllte. Eine Glasampulle mit 20 ml Monomerflüssigkeit wurde in die Vorrichtung eingesetzt. Der Ampullenkopf wurde abgebrochen und ein Temperaturfühler der Temperaturmesseinheit testo 725-2 der Firma Testo wurde eingesetzt. Danach wurden 90 ml Wasser in die Vorrichtung gegeben. Es setzte sofort nach der Zugabe des Wassers eine Wärmeentwicklung ein. Die Temperatur der Monomerflüssigkeit in der Glasampulle wurde ab Zugabe des Wassers minütlich gemessen. Die Ergebnisse sind in Tabelle 3 angegeben.

**Tabelle 3**

| | | | | | | | | | | |
|---|---|---|---|---|---|---|---|---|---|---|
| Zeit [min] | 1 | 2 | 3 | 4 | 5 | 6 | 7 | 8 | 9 | 10 |
| Temperatur [°C] | 63,5 | 67,8 | 70,8 | 71,6 | 72,2 | 70,6 | 69,0 | 68,0 | 67,0 | 65,9 |

Es ist ersichtlich, dass eine Temperatur oberhalb von 60 °C bereits nach einer Minute erreicht ist und für einen Zeitraum von mindestens 10 Minuten gehalten wird. Von Minute 3 bis Minute 6 wurde eine Temperatur von mindestens 70 °C Minuten erreicht. Die anschließende Abkühlung erfolgt nur langsam.

### Beispiel 4 - Bestimmung der Erwärmung 2

In einem weiteren Versuch auf der Basis des oben beschriebenen Versuchs wurden 65 g getrocknetes Zeolith A mit 65 ml Wasser in der Vorrichtung miteinander vermischt. Es setzte sofort nach der Zugabe des Wassers eine Wärmeentwicklung ein. Die Ergebnisse sind in Tabelle 4 dargestellt.

**Tabelle 4**

| | | | | | | | | | | |
|---|---|---|---|---|---|---|---|---|---|---|
| Zeit [min] | 1 | 2 | 3 | 4 | 5 | 6 | 7 | 8 | 9 | 10 |
| Temperatur [°C] | 40,0 | 48,0 | 52,0 | 51,0 | 52,0 | 52,0 | 52,0 | 52,0 | 52,0 | 52,0 |

Die Temperaturen sind niedriger. Eine Temperatur oberhalb von 50 °C ist nach 3 Minuten erreicht und wird für einen Zeitraum von mindestens 8 Minuten gehalten. In der hier untersuchten Konstellation erfolgte keine Abkühlung in den ersten 10 Minuten; die Temperatur blieb ab Minute 5 konstant.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: eine schematische Darstellung einer Vorrichtung,
- Figur 2:: Schritte eines Verfahrens zur Herstellung von Knochenzement,
- Figur 3:: eine perspektivische Darstellung einer Vorrichtung,
- Figuren 5 bis 8:: Schnittzeichnungen einer Vorrichtung bei der Verwendung,
- Figur 9:: eine Schnittzeichnung einer weiteren Vorrichtung,
- Figuren 10 und 12:: vergrößerte Details,
- Figur 11:: eine perspektivische Darstellung einer Vorrichtung, sowie
- Figuren 13 bis 16:: Zeichnungen einer Vorrichtung bei der Verwendung.

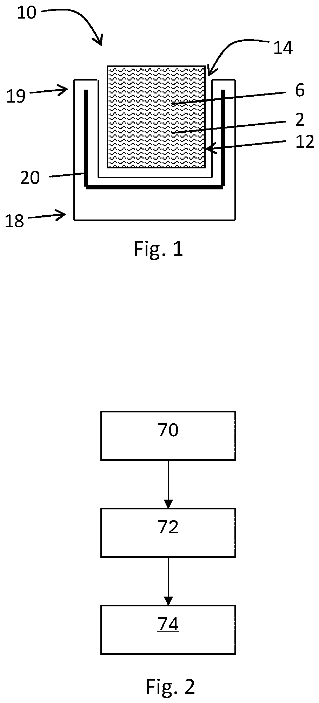

Figur 1 zeigt eine einfache erfindungsgemäße Vorrichtung 10 zur Erwärmung einer Komponente 2 zur Herstellung von Knochenzement. Die Vorrichtung 10 umfasst einen Erwärmungsraum 12, der mittels einer Wärmequelle 20 erwärmt werden kann. Typischerweise sind der Erwärmungsraum 12 und die Wärmequelle 20 in einem Hauptkörper der Vorrichtung 10 angeordnet. Eine Unterseite 18 der Vorrichtung ist zum Aufstellen auf einen Grund eingerichtet. Insbesondere ist der Erwärmungsraum 12 von oben zugänglich. Es befindet sich ein Zugang 14 zum Erwärmungsraum 12 insbesondere an der Oberseite 19. Durch den Zugang 14 kann ein Behälter 6 mit der darin befindlichen Komponente 2 in den Erwärmungsraum eingebracht werden.

Figur 2 zeigt Schritte eines Verfahrens. Zunächst erfolgt ein Erwärmen 70 mindestens einer Komponente zur Herstellung von Knochenzement. Anschließend erfolgt ein Inkontaktbringen und Mischen 72 der Komponente mit zumindest einer weiteren Komponente. Auf diese Weise wird Knochenzement 74 hergestellt. Durch das vorherige Erwärmen 70 erfolgt die Aushärtung schneller als bei herkömmlichen Verfahren.

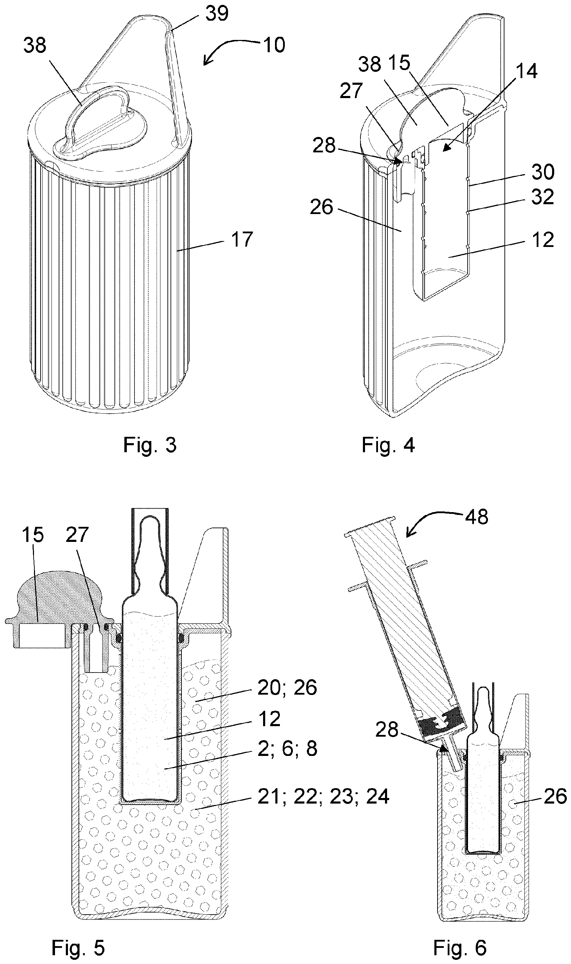

Die Figuren 3 und 4 zeigen ein weiteres Ausführungsbeispiel einer Vorrichtung 10 zum Erwärmen einer Komponente 2 zur Herstellung von Knochenzement. Die Vorrichtung 10 umfasst einen Erwärmungsraum 12 zur Aufnahme der Komponente bzw. eines Behälters mit der Komponente, der mit einem Zugang 14 von oben zugänglich ist. Die Vorrichtung 10 umfasst ferner einen Raum 26 für einen Wärmespeicher als Wärmequelle, insbesondere einen thermochemischen Wärmespeicher. Der Wärmespeicher umfasst ein hier nicht dargestelltes Wärmespeichermedium, das nach Aktivierung mittels Zufügen einer Flüssigkeit Wärme abgibt. Der Raum 26 umfasst zu diesem Zweck eine Öffnung 28, durch die die Flüssigkeit eingefüllt werden kann.

Die Öffnung 28 ist durch einen Deckel 27 verschlossen. Der Zugang 14 ist durch eine Abdeckung 15 verschlossen. In der hier gezeigten Ausführungsform sind der Deckel 27 und die Abdeckung 15 als Einheit ausgeführt und durch einen gemeinsamen Griff 38 miteinander verbunden. Auf diese Weise können sie gemeinsam geöffnet und verschlossen werden.

Die Wandung 30, die den Erwärmungsraum 12 von dem Raum 26 des Wärmespeichers abgrenzt, weist eine Vielzahl an Durchgangslöchern auf. So kann Flüssigkeit aus dem Wärmespeicher in den Erwärmungsraum 12 gelangen, um den Behälter zu benetzen und die Komponente beschleunigt zu erwärmen.

Die Außenwand 17 ist geriffelt, um den Halt beim manuellen Greifen zu verbessern und die Kontaktfläche zur Hand verringern. Auf diese Weise wird die Gefahr von Verbrennungen reduziert. Die Vorrichtung 10 umfasst einen Ausguss 39, um die erwärmte Komponente gezielt auszugießen.

Die Figuren 5 bis 8 zeigen Schritte der Verwendung der Vorrichtung 10, insbesondere der Vorrichtung 10 aus den Figuren 3 und 4. Figur 5 zeigt einen Zustand, in dem als Wärmequelle 20 ein Wärmespeicher 21, nämlich ein thermochemischer Wärmespeicher 22 vorhanden ist. Es befindet sich in dem Raum 26 ein Wärmespeichermedium 23, beispielsweise Partikel 24 aus Zeolith. In den Erwärmungsraum 12 wurde ein Behälter 6, nämlich eine Ampulle 8 eingesetzt. Die Ampulle 8 enthält eine Komponente 2 zur Herstellung von Knochenzement, insbesondere eine flüssige Monomerkomponente.

Die Einheit aus Deckel 27 und Abdeckung 15 weist in dieser Ausführungsform im Bereich des Deckels 27 längere Leitelemente auf als im Bereich der Abdeckung 15. Dies ermöglicht, die Einheit nach oben in axialer Richtung teilweise herauszuziehen, so dass die Abdeckung 15 gelöst ist, aber der Deckel 27 noch zumindest teilweise fixiert ist. Anschließend wurde die Einheit um eine vertikal ausgerichtete Achse um 180° gedreht und wieder nach unten geschoben, um in die gezeigte Position zu gelangen. So konnte die Ampulle 8 eingesetzt werden, während der Raum 26 weiterhin verschlossen blieb. Ein verfrühtes Aktivieren der Wärmequelle 20 und/oder ein Herausfallen von Wärmespeichermedium 23 konnte so verhindert werden.

Figur 6 zeigt einen Zustand, in dem die Einheit aus Deckel 27 und Abdeckung 15 vollständig entfernt worden ist und über die Öffnung, beispielhaft hier mit einer Spritze 48, eine Flüssigkeit wie Wasser in den Raum 26 gefüllt wird. Auf diese Weise wird die Wärmequelle aktiviert und die Komponente wird erwärmt. Der Deckel 27 kann wieder aufgesetzt werden, um ein Austreten von Wasser und/oder Wärmespeichermedium zu verhindern.

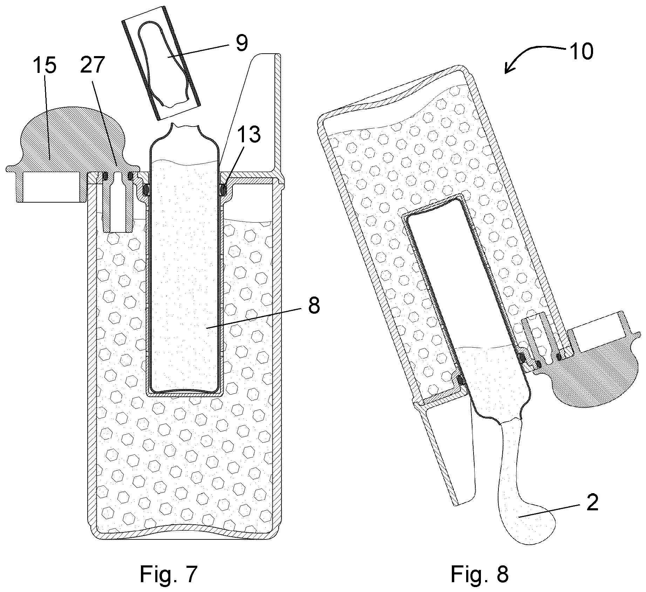

Nach wenigen Minuten ist die Erwärmung abgeschlossen. Der Kopf 9 der Kartusche kann abgebrochen werden, wie in Figur 7 gezeigt. Anschließend kann, wie in Figur 8 dargestellt, die gesamte Vorrichtung 10 gekippt werden, um die erwärmte Komponente 10 ausfließen zu lassen. Dies wird unter anderem ermöglicht durch das Dichtungselement 13, das den Zugang gegenüber der Ampulle 8 abdichtet, wie in Figur 7 gezeigt, und die Ampulle 8 klemmend hält.

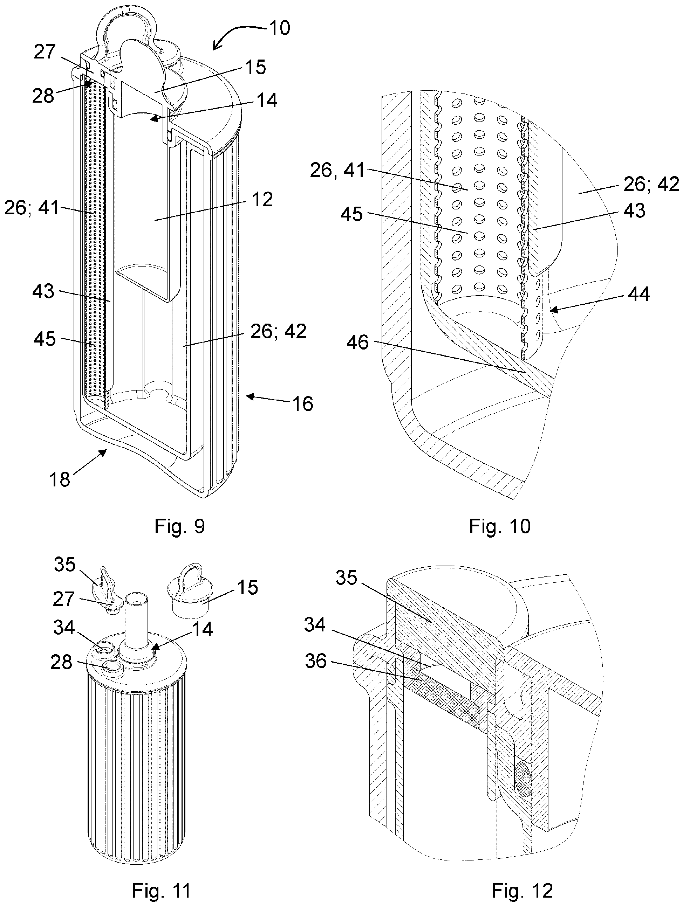

Die Figuren 9 bis 13 zeigen eine weiterentwickelte Vorrichtung 10, die auf der Vorrichtung der Figuren 3 bis 8 basiert. Im Folgenden wird lediglich auf die Unterschiede eingegangen. Es sind an unterschiedlichen Komponenten Änderungen durchgeführt worden, die erfindungsgemäß jeweils unabhängig voneinander realisiert werden können oder nicht.

Zum einen ist, wie in Figur 9 ersichtlich ist, die umlaufende Außenfläche 16, bei der hier gezeigten kreiszylinderförmigen Grundform also die Mantelfläche der Vorrichtung thermisch isoliert. Dies ist beispielhaft dadurch realisiert, dass eine Doppelwand vorgesehen ist. In einem Zwischenraum zwischen den einzelnen Schichten der Doppelwand befindet sich Luft, ein Vakuum und/oder ein Isolationsmaterial wie ein Kunststoffschaum. Im hier gezeigten Ausführungsbeispiel ist auch die Unterseite 18 auf diese Weise thermisch isoliert. Der Zwischenraum an Außenseite 16 und Unterseite 18 ist wie bei einer Thermoskanne durchgehend ausgeführt.

Zudem ist der Raum 26 des thermochemischen Wärmespeichers in zwei Bereiche 41, 42 unterteilt, die durch eine Wand 43 voneinander getrennt sind und lediglich im Bereich der unteren Begrenzung 46 des Raums 26 miteinander verbunden sind. Das eingefüllte Wasser muss daher zunächst den ersten Bereich 41 durchströmen, bevor es im unteren Bereich des Raums 26 in den zweiten Bereich 42 gelangen kann. Der erste Bereich 41 ist leer bzw. mit Luft gefüllt, während der zweite Bereich 42 das hier nicht dargestellte Wärmespeichermedium enthält. Das Einfüllen des Wassers kann demnach einfach und rasch erfolgen und das Wärmespeichermedium kommt erst im zweiten Bereich 42 in Kontakt mit dem Wasser. So wird ein Ausströmen von entstehendem Wasserdampf durch die Öffnung 28 verhindert.

Die unten befindliche Verbindung 44 zwischen dem ersten Bereich 41 und dem zweiten Bereich 42 ist vergrößert in Figur 10 dargestellt. Ein erster Bereich 41 ist hier beispielhaft röhrenförmig und erstreckt sich von der Öffnung 26 ausgehend insbesondere geradlinig nach unten. Es ist ersichtlich, dass im ersten Bereich 41 hier beispielhaft ein perforiertes Einlaufelement 45 in Form eines Korbes angeordnet ist. Das Einlaufelement 45 verhindert das Eindringen des Wärmespeichermediums in den ersten Bereich 41. Die Wand 43 ist unten im Bereich der Verbindung 44 offen bzw. nicht vorhanden. Die Verbindung grenzt im hier gezeigten Ausführungsbeispiel direkt an die untere Begrenzung 46 des Raums 26 an.

Ferner ist die Abdeckung 15 separat vom Deckel 27 ausgeführt. Es ist jedoch zusätzlich eine Entlüftungsöffnung 34 vorgesehen, die den Raum 26 mit der Umgebung verbindet. Der Deckel 27 ist beispielhaft als Einheit mit einem Deckel 35 der Entlüftungsöffnung 34 ausgeführt. Die Einheit hat einen gemeinsamen Griff.

Wie in der vergrößerten Darstellung in Figur 12 ersichtlich ist, ist die Entlüftungsöffnung 34 mit einem Filterelement 36 verschlossen, das Gase durchlässt und Feststoffe und/oder Flüssigkeiten zurückhält.

Darüber hinaus ist ein Trichter 49 vorhanden, der dem Abbrechen des Kopfes der Ampulle 8 und/oder dem gezielten Ausgießen der Komponente aus der Ampulle 8 dient. Der Trichter 49 kann über den Zugang 14 gestülpt und/oder im Bereich des Zugangs 14 befestigt werden.

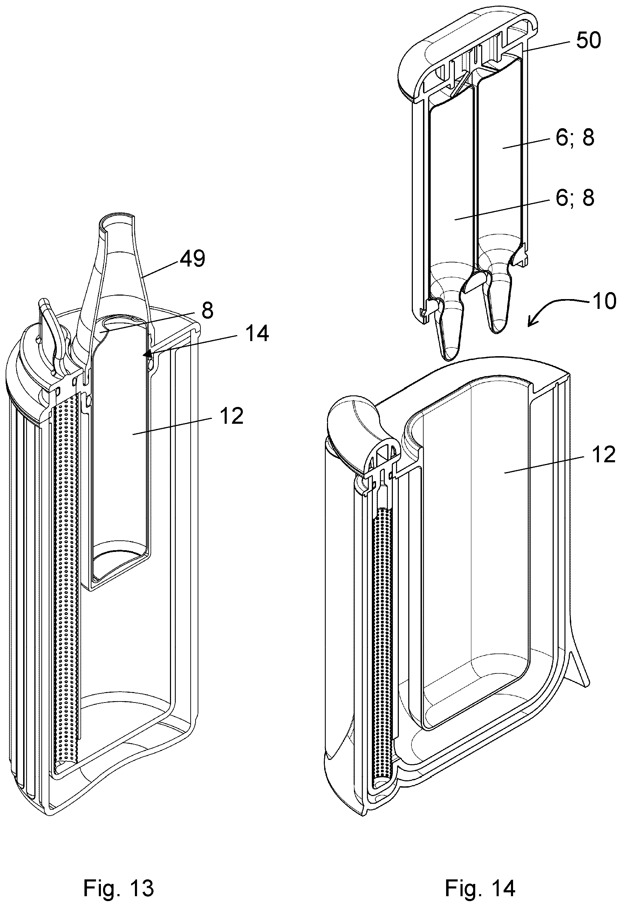

Die Figuren 14 bis 16 zeigen eine weiterentwickelte Vorrichtung 10, die auf der Vorrichtung der Figuren 9 bis 13 basiert. Im Folgenden wird lediglich auf Unterschiede eingegangen. Es sind an unterschiedlichen Komponenten Änderungen durchgeführt worden, die erfindungsgemäß jeweils unabhängig voneinander realisiert werden können oder nicht.

Die Vorrichtung 10 ist dazu eingerichtet zwei oder mehr Behälter 6, insbesondere in Form von Ampullen 8, nebeneinander aufzunehmen. Dazu kann die Vorrichtung 10 im Querschnitt langgestreckt sein. Darüber hinaus ist eine Halteeinrichtung 50 vorhanden, die die zwei oder mehr Behälter 6 hält. Die Halteeinrichtung 50 kann mit den Behältern in den Erwärmungsraum 12 eingesetzt werden. Die Vorrichtung 10 kann die Halteeinrichtung 50 umfassen.

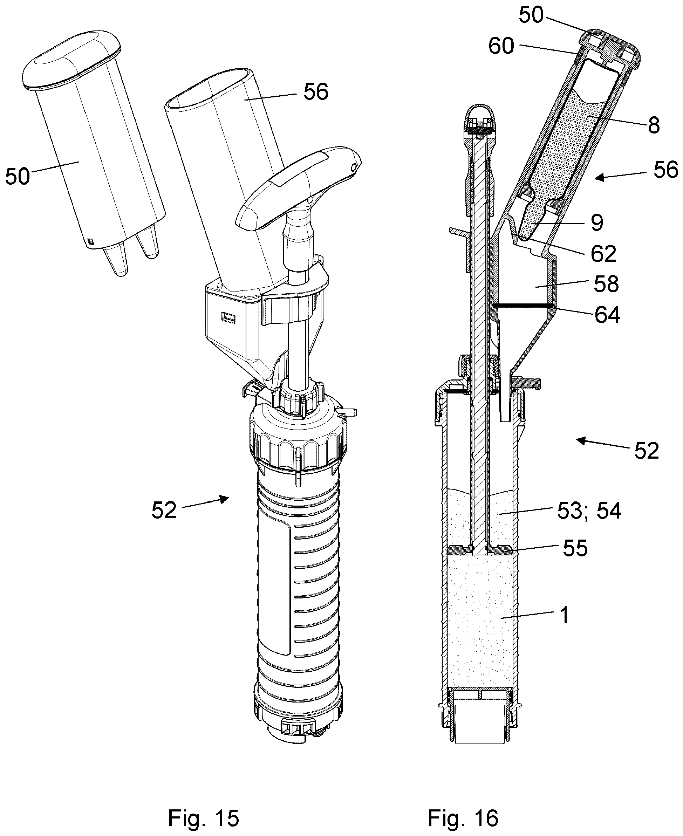

Die Halteeinrichtung 50 mit den Behältern 6 kann, wie in den Figuren 15 und 16 gezeigt, mechanisch mit einer Mischvorrichtung 52 gekoppelt werden. Insbesondere umfasst die Mischvorrichtung 52 einen Mischbehälter 53 mit einem Innenraum 54 zum Mischen von Knochenzement. Der Mischbehälter 53 ist hier als Kartusche ausgeführt. Im Innenraum 54 ist ein Mischelement 55 angeordnet, beispielsweise ein Mischteller, welches entlang der Längsachse des Mischbehälters 53 manuell in Innenraum 54 hin- und herbewegt werden kann, um durch Mischen der Komponenten Knochenzement herzustellen. Die zweite Komponente, in der hier gezeigten Ausführungsform eine Pulverkomponente bzw. ein PMMA-Pulver, kann im Innenraum 54 gelagert werden.

Die Halteeinrichtung 50 wird hier mechanisch mit einer Öffnungsvorrichtung 56 gekoppelt, die mechanisch mit der Mischvorrichtung 52 verbunden ist und/oder Teil der Mischvorrichtung 52 ist. Die Öffnungsvorrichtung 56 bildet einen Hohlraum aus, in den die Halteeinrichtung 50 mit den Behältern entlang der Längsrichtung eingeführt werden kann. Der eingeführte Zustand ist in Figur 16 gezeigt. Die Ampullen 8 sind mit dem Kopf 9 nach unten ausgerichtet. Nahe des Kopfes 9 befindet sich ein beispielsweise keilförmiges Scherelement 62. Die Halteeinrichtung 50 kann entlang der Längsachse im Hohlraum 56 der Öffnungsvorrichtung 56 bewegt werden. Ein Sicherungsclip 60 verhindert ein weiteres Einschieben der Halteeinrichtung 50.

Der Sicherungsclip 60 kann abgezogen bzw. entfernt werden, bezogen auf Figur 16 aus der Bildebene heraus. Auf diese Weise wird das Einschieben der Halteeinrichtung 50 in die Öffnungsvorrichtung 56 freigegeben. Durch manuellen, axial ausgerichteten Druck auf die Halteeinrichtung 50 bzw. die Ampulle 8 wird der Kopf 9 der Ampulle 8 schräg gegen das Scherelement 61 gedrückt und auf diese Weise abgebrochen.

Zwischen dem Hohlraum und dem Mischbehälter 53 ist ein Fluidkanal 58 angeordnet, in dem die erwärmte Monomerflüssigkeit aus der Ampulle 8 verlustfrei in den Innenraum 54 strömen kann, insbesondere schwerkraftbedingt. Der Fluidkanal 58 kann einen trichterförmigen Bereich aufweisen. Im Fluidkanal 58 befindet sich bevorzugt ein Filter 64, der möglicherweise entstehende Glasstückchen zurückhält. Der Fluidkanal 58 kann am unteren Ende ein Einleitungsrohr zum Einleiten der Flüssigkeit in den Innenraum 54 aufweisen. Das Einleitungsrohr kann so ausgebildet sein, dass es nach dem Einleiten herausgezogen werden kann. Es kann ein Verschluss, beispielsweise ein Schieber, am Mischbehälter 53 vorgesehen sein, z. B. im Kopf der Kartusche, um anschließend den Mischbehälter 53 zu verschließen.

Wenn die Monomerflüssigkeit im Innenraum 54 ist, kann durch manuelles Mischen wie beschrieben und/oder unter Einwirkung eines Vakuums der Knochenzement hergestellt werden. Anschließend erfolgt ein Austragen des Knochenzements, beispielsweise durch Auspressen, z. B. mittels einer Auspressvorrichtung und/oder beschleunigt durch ein Vakuum.

**Bezugszeichenliste**

| | |
|---|---|
| Komponente | 1 |
| Komponente | 2 |
| Behälter | 6 |
| Ampulle | 8 |
| Kopf | 9 |
| Vorrichtung | 10 |
| Erwärmungsraum | 12 |
| Dichtungselement | 13 |
| Zugang | 14 |
| Abdeckung | 15 |
| Außenseite | 16 |
| Außenwand | 17 |
| Unterseite | 18 |
| Oberseite | 19 |
| Wärmequelle | 20 |
| Wärmespeicher | 21 |
| Thermochemischer Wärmespeicher | 22 |
| Wärmespeichermedium | 23 |
| Partikel | 24 |
| Raum | 26 |
| Deckel | 27 |
| Öffnung | 28 |
| Wasser | 29 |
| Wandung | 30 |
| Durchgangsöffnung | 32 |
| Entlüftungsöffnung | 34 |
| Deckel | 35 |
| Filterelement | 36 |
| Griff | 38 |
| Ausguss | 39 |
| Erster Bereich | 41 |
| Zweiter Bereich | 42 |
| Wand | 43 |
| Verbindung | 44 |
| Einlaufelement | 45 |
| Untere Begrenzung | 46 |
| Spritze | 48 |
| Trichter | 49 |
| Halteeinrichtung | 50 |
| Mischvorrichtung | 52 |
| Mischbehälter | 53 |
| Innenraum | 54 |
| Mischelement | 55 |
| Öffnungsvorrichtung | 56 |
| Fluidkanal | 58 |
| Sicherungsclip | 60 |
| Scherelement | 62 |
| Filter | 64 |
| Erwärmen | 70 |
| Inkontaktbringen und Mischen | 72 |
| Knochenzement | 74 |

## Patentansprüche

1. Verfahren zur Herstellung von Knochenzement, bei dem zwei Komponenten (1, 2) miteinander in Kontakt gebracht und gemischt werden, um Knochenzement herzustellen, **dadurch gekennzeichnet, dass** mindestens eine Komponente (2) der zwei Komponenten (1, 2) oder der hergestellte Knochenzement erwärmt wird.

2. Verfahren nach dem vorhergehenden Anspruch, **dadurch gekennzeichnet, dass** sich ein Behälter (6), der eine Komponente (2) enthält, während des Erwärmens in einem Erwärmungsraum (12) befindet.

3. Verfahren nach dem vorhergehenden Anspruch, **dadurch gekennzeichnet, dass** der Behälter (6) von einer Halteeinrichtung (50) im Erwärmungsraum (12) gehalten wird, wobei die Halteeinrichtung (50) mit dem Behälter (6) nach dem Erwärmen aus dem Erwärmungsraum (12) entnommen und mechanisch mit einer Mischvorrichtung (52) gekoppelt wird.

4. Vorrichtung (10) zur Erwärmung einer Komponente (2) zur Herstellung von Knochenzement, umfassend einen Erwärmungsraum (12) zur Aufnahme der Komponente (2) sowie eine Wärmequelle (20) zum Erwärmen der in dem Erwärmungsraum (12) befindlichen Komponente (2).

5. Vorrichtung (10) nach dem vorhergehenden Anspruch, wobei die Wärmequelle (20) einen Wärmespeicher (21) umfasst, der nach Aktivierung Wärme abgeben kann, insbesondere einen thermochemischen Wärmespeicher (22).

6. Vorrichtung (10) nach dem vorhergehenden Anspruch, **dadurch gekennzeichnet, dass** der thermochemische Wärmespeicher (22) so beschaffen ist, dass er durch Zugabe von Wasser (29) aktiviert werden kann,
und/oder dass der thermochemische Wärmespeicher (22) Zeolith, Kieselgel und/oder wasserfreies Calciumchlorid umfasst.

7. Vorrichtung (10) nach dem vorhergehenden Anspruch, wobei der thermochemische Wärmespeicher (22) ein Wärmespeichermedium (23) aufweist, welches in Form von Partikeln (24) mit einem Durchmesser von mindestens 0,1 mm und/oder höchstens 4 mm vorliegt.

8. Vorrichtung (10) nach einem der vier vorhergehenden Ansprüche, ferner umfassend einen Temperaturindikator zur optischen Anzeige einer definierten Temperatur im Inneren der Vorrichtung (10).

9. Vorrichtung (10) nach einem der fünf vorhergehenden Ansprüche, wobei zumindest eine umlaufende Außenseite (16) der Vorrichtung (10) thermisch isoliert ist.

10. Vorrichtung (10) nach einem der sechs vorhergehenden Ansprüche, wobei eine Wandung, die den thermochemischen Wärmespeicher (22) von dem Erwärmungsraum (12) trennt, eine oder mehrere Durchgangsöffnungen (32) aufweist.

11. Vorrichtung (10) nach einem der sieben vorhergehenden Ansprüche, wobei eine Außenwand (17) der Vorrichtung (10) im Bereich des thermochemischen Wärmespeichers (22) zumindest bereichsweise transparent oder transluzent ist.

12. Vorrichtung (10) nach einem der sechs vorhergehenden Ansprüche, wobei der thermochemische Wärmespeicher (22) eine Entlüftungsöffnung (36) aufweist, um beim Zugeben von Wasser (29) verdrängte Luft ausströmen zu lassen.

13. Vorrichtung (10) nach einem der sieben vorhergehenden Ansprüche, wobei ein Raum (26) des thermochemischen Wärmespeichers (22) zwei Bereiche (41, 42) aufweist, wobei ein erster Bereich (41) zum Zugeben des Wassers (29) dient und ein zweiter Bereich (42) ein Wärmespeichermedium enthält, wobei der erste Bereich (41) und der zweite Bereich (42) lediglich im Bereich einer unteren Begrenzung (46) des Raums (26) miteinander verbunden sind.

14. Vorrichtung (10) nach einem der zehn vorhergehenden Ansprüche, wobei die Vorrichtung (10) eine Halteeinrichtung (50) zum Halten des Behälters (6) im Erwärmungsraum (12) aufweist, wobei die Halteeinrichtung (50) zum mechanischen Koppeln mit einer Mischvorrichtung (52) eingerichtet ist.

15. Vorrichtung (10) nach einem der elf vorhergehenden Ansprüche, wobei ein Gehäuse der Vorrichtung (10) zumindest im Wesentlichen durch Kunststoffspritzgießen hergestellt ist.
